(19) Europäisches Patentamt
European Patent Office
Office européen des brevets

(11) Veröffentlichungsnummer: **0 140 251**
**B2**

(12) # NEUE EUROPÄISCHE PATENTSCHRIFT

(45) Veröffentlichungstag der neuen Patentschrift: **06.09.89**

(51) Int. Cl.⁴: **C 07 C 143/46**, C 07 C 139/00

(21) Anmeldenummer: **84112279.9**

(22) Anmeldetag: **12.10.84**

(54) **Verfahren zur Herstellung von Alkali- und Erdalkalisalzen von Acyloxibenzolsulfonsäuren.**

(30) Priorität: **19.10.83 DE 3337921**

(43) Veröffentlichungstag der Anmeldung: **08.05.85 Patentblatt 85/19**

(45) Bekanntmachung des Hinweises auf die Patenterteilung: **11.06.86 Patentblatt 86/24**

(45) Bekanntmachung des Hinweises auf die Entscheidung über den Einspruch: **06.09.89 Patentblatt 89/36**

(84) Benannte Vertragsstaaten: **AT BE CH DE FR GB IT LI NL SE**

(56) Entgegenhaltungen:
**DE-A- 859 451**
**DE-A- 2 602 510**

**L. Habicht, Int. Kongr.f.grenzfl.akt. Stoffe, Köln 1960, Untergruppe A/I/3, S. 116-118**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Balzer, Wolf-Dieter, Dr., Bruesseler Ring 34, D-6700 Ludwigshafen (DE)**
Erfinder: **Bechtolsheimer, Hans-Heinrich, Dr., Ringstrasse 7, D-6521 Dittelsheim-Hessloch (DE)**
Erfinder: **Beyer, Karl-Heinz, Dr., Knietschstrasse 6, D-6710 Frankenthal (DE)**
Erfinder: **Fikentscher, Rolf, Dr., Von-Stephan-Strasse 27, D-6700 Ludwigshafen (DE)**
Erfinder: **Perner, Johannes, Dr., Ginsterweg 4, D-6730 Neustadt (DE)**
Erfinder: **Widder, Rudi, Dr., In der Taesch 7, D-6906 Leimen (DE)**
Erfinder: **Wolf, Helmut, Im Zollstock 6, D-6733 Hassloch (DE)**

**EP 0 140 251 B2**

**Beschreibung**

Die Erfindung betrifft die Herstellung der Alkali- und Erdalkalisalze von Acyloxibenzolsulfonsäuren durch Neutralisation. Diese Salze können insbesondere als Acylierungsmittel in wäßrigen Medien oder als Waschmittelkomponente verwendet werden.

Es ist bekannt, daß Acyloxibenzolsulfonsäuren als aktivierte Ester Acylierungsmittel für Amine, Mercaptane, Wasserstoffperoxid und andere Verbindungen mit aktivem Wasserstoff sind. Für manche Anwendungen, wie die Acylierung von Feststoffen oder von wasserunlöslichen polymeren Verbindungen oder bei der Anwendung in Waschmitteln als Kalkbleichaktivatoren, beispielsweise gemäß EP-Anmeldung 28 432, GB-PS 864 798 oder der DE-OS 2 602 510, sind wasserlösliche Acylierungsmittel zweckmäßig. Solche wasserlöslichen Acylierungsmittel sind beispielsweise die Salze von Acyloxibenzolsulfonsäuren, wie die bekannten Benzoyl- oder Acetyl-p-oxibenzolsulfonate. Weiterhin geht beispielsweise aus der US-PS 3 503 888 die Verwendung von Salzen von Acyloxibenzolsulfonsäuren in Toilettenseifen hervor.

Die Acyloxibenzolsulfonate können beispielsweise durch Umsetzung von Natriumphenolsulfonat mit einem Säurechlorid oder -anhydrid direkt oder in einem inerten organischen Medium in suspendierter Form hergestellt werden. Diese Umsetzung verläuft in der Regel sehr langsam, da die Reaktionspartner bzw. die Reaktionsprodukte keine homogene Phase bilden. Man muß, um einigermaßen günstige Ausbeuten zu erhalten, mit einem Überschuß an Acylierungsmittel und bei erhöhten Temperaturen, wie beispielsweise in der DE-OS 2 602 510 beschrieben wird, arbeiten.

Ein weiterer Nachteil dieses Verfahrens liegt darin, daß das Natriumsalz der zu veresternden Phenolsulfonsäure mit Kristallwasser kristallisiert und praktisch nur so erhältlich ist. Eine Entwässerung des Natriumphenolsulfonats unter üblichen technischen Bedingungen, wie Erhitzen unter vermindertem Druck oder azeotropes Entwässern, ist aufwendig, teuer und kann zu Verfärbungen führen. Wird kristallwasserhaltiges Natriumphenolsulfonat mit einem Carbonsäurechlorid acyliert, dann kann sich durch das Kristallwasser in größerer Menge schwer abtrennbare Säure bilden.

Es ist bekannt, daß die obengenannten aktivierten Phenolester sehr hydrolyseempfindlich sind. In Wasser tritt schon bei Raumtemperaturen nach kurzer Zeit weitgehend Hydrolyse ein. Auch bei pH-Werten oberhalb 7,5, d.h. im alkalischen Bereich, tritt sehr rasch Verseifung ein, was beispielsweise der US-PS 3 503 888, Spalte 4, Zeilen 3ff, entnommen werden kann. Aus diesem Grunde lassen sich die Alkali- und Erdalkalisalze von Acyloxibenzolsulfonsäuren ohne Verlust durch Verseifungsreaktionen nur sehr schwer herstellen. Auch Versuche, die wasserfreien Acyloxibenzolsulfonsäuren in einem inerten organischen Lösungsmittel, wie Dioxan, Aceton oder chlorierten Kohlenwasserstoffen, mit z.B. Alkalicarbonat, -bicarbonat oder -acetat in Salze zu überführen, führten nicht zum Erfolg. Protische Lösungsmittel, insbesondere niedere Alkohole oder Glykole, können als Lösungsmittel nicht verwendet werden, da mit ihnen Umesterung eintritt. Aus diesem Grunde sind die Salze der am häufigsten verwendeten Acyloxibenzolsulfonsäuren in reiner Form nur schwierig zugänglich.

Aufgabe der Erfindung ist es, ein in großtechnischem Maßstab leicht durchzuführendes Verfahren zur Herstellung von Alkali- und Erdalkalisalzen von Acyloxibenzolsulfonsäuren durch Neutralisation in wäßrigem Medium zu entwickeln, um für die praktische Verwendung möglichst reine und nicht gefärbte Salze zur Verfügung zu haben.

Gegenstand der Erfindung ist ein Verfahren zur Herstellung der Alkali- und Erdalkalisalze von Acyloxibenzolsulfonsäuren der Formel I

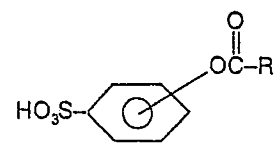
(I),

in der R einen gesättigten oder ungesättigten Alkylrest mit 1 bis 17 C-Atomen, einen Phenylrest, der gegebenenfalls ein- bis zweifach durch einen Alkylrest mit 1 bis 3 C-Atomen, ein Halogenatom, eine Methoxy- oder Nitrogruppe substituiert ist, bedeutet, durch Neutralisation, bei dem man eine flüssige Acyloxibenzolsulfonsäure der Formel (I) mit einer wäßrigen Lösung von Alkali oder Erdalkali in Form des Hydroxids, Carbonats oder Bicarbonats in Wasser bei Temperaturen von 0 bis 60 °C unter guter Durchmischung so zusammenbringt, daß ein pH-Wertbereich von 3,0 bis 5,5 eingehalten wird, und man gegebenenfalls das erhaltene Salz in an sich üblicher Weise aus der wäßrigen Lösung isoliert.

Als Reste für R kommen gesättigte und ungesättigte, geradkettige oder verzweigte Alkylreste mit 1 bis 17 C-Atomen, bevorzugt 5 bis 17 C-Atomen, in Betracht. Im einzelnen seien beispielsweise genannt Methyl, Ethyl, Pentyl, n-Heptyl, 2-Ethylpentyl, 2-Ethylhexyl, n-Octyl, verzweigte Octylreste, 3,5,5-Trimethylpentyl, n-Nonanyl, 3,5,5-Trimethylhexyl, Undecyl, Heptadecyl und Heptadecenyl.

Substituierte Phenylreste für R sind beispielsweise Tolyl, Anisyl und Chorphenyl.

Im übrigen steht in der Formel (I) der Rest –O–COR bevorzugt in p-Stellung.

Die Herstellung der Acyloxibenzolsulfonsäuren der Formel (I) erfolgt in bekannter Weise, wie sie beispielsweise in der US-PS 3 503 888 beschrieben wird.

Durch das Verfahren der Erfindung können unter den speziellen Bedingungen in nicht vorhersehbarer Weise Acyloxibenzolsulfonsäuren in wäßrigem Medium, ohne daß nennenswerte Verseifung eintritt, neutralisiert werden. Zweckmäßig geht man so vor, daß man in Wasser gleichzeitig die flüssige Acyloxibenzolsulfonsäure und eine 5 bis 50 gew.%ige wäßrige Lösung von Alkali- oder Erdalkali in Form des Hydroxids, Carbonats oder Bicarbonats bei Temperaturen von 0 bis 60 °C,

bevorzugt 10 bis 40 °C, unter Rühren so einlaufen läßt, daß ein pH-Wertbereich von 3,0 bis 5,5 eingehalten wird.

Die Neutralisation kann diskontinuierlich oder kontinuierlich durchgeführt werden. Bei der kontinuierlichen Arbeitsweise werden die Komponenten Wasser, Acyloxibenzolsulfonsäure und Alkali in einem statischen oder dynamischen Mischer zusammengeführt.

Es wird darauf hingewiesen, daß beim Lösen der Acyloxibenzolsulfonsäuren in Wasser eine beträchtliche Wärmemenge freigesetzt wird. Dazu kommt noch die Neutralisationswärme. Diese gesamte Wärmemenge muß durch Kühlung abgeführt werden. Das führt dazu, daß bei der technischen Durchführung der erfindungsgemäßen Neutralisation in der Regel Zeiten von 3 bis 10 Stunden in Abhängigkeit von der zu neutralisierenden Menge Acyloxibenzolsulfonsäure erforderlich sind. Führt man die Neutralisation kontinuierlich durch, so muß man einen entsprechend dimensionierten Wärmeaustauscher dem Mischer nachschalten.

Durch das erfindungsgemäße Verfahren können stabile wäßrige Lösungen der Acyloxibenzolsulfonate in Konzentrationen von 20 bis 60 Gew.% hergestellt werden. Aus diesen Lösungen können die reinen Salze in an sich üblicher Weise, beispielsweise durch Eindampfen, Sprühtrocknen, Gefriertrocknen, Walzentrocknung oder Wirbelbettrocknen, isoliert werden.

In einer besonders bevorzugten Ausführungsform wird die erfindungsgemäße Neutralisation in Gegenwart von 1 bis 2 Gew.%, bezogen auf die Acyloxibenzolsulfonsäure, von einem wasserlöslichen Phosphat, Phosphit, Tartrat oder einem Komplexbildner für Schwermetalle oder einem Polymer aus Acrylsäure und Maleinsäure durchgeführt. Dabei werden in der Regel die wasserlöslichen Natriumsalze verwendet.

Löst man die durch Sprühtrocknung gewonnenen Natriumsalze der Acyloxibenzolsulfonsäure in Wasser, so erhält man gelbe bis schwach bräunliche Lösungen, die am Licht nachdunkeln können. Überraschenderweise wurde gefunden, daß diese Lösungen deutlich weniger gefärbt sind und bei der Weiterverarbeitung weniger zu Verfärbungen neigen, wenn man die Neutralisation in Gegenwart der obengenannten Verbindungen durchführt. Als Beispiele für derart wirksame Substanzen seien genannt: Natriumdihydrogenphosphat, Dinatriumtartrat, Natriumhydrogentartrat, Natriumphosphit, Unterphosphorige Säure, Nitrilotriessigsäure, Ethylendiamintetraessigsäure, Diethylentriaminpentaessigsäure, Hydroxiethylethylendiamintriessigsäure, Nitrilotrimethylenphosphonsäure oder Polycarbonsäuren aus Acrylsäure und/oder Maleinsäure und ihre Na-Salze.

Beispiel 1

100 Teile einer Acyloxibenzolsulfonsäure der Formel I läßt man unter gutem Rühren zu 100 Teilen Wasser zulaufen. Gleichzeitig wird 50 gew.%ige wäßrige Natronlauge so zugetropft, daß sich in der wäßrigen Lösung ein pH-Wert von 3,0 bis 5,5 (Verfolgung mittels Glaselektrode) einstellt. Die Temperatur der Reaktionsmischung wird durch Kühlung unter 50 °C gehalten. Nach dem Ende der Zugabe der Acyloxibenzolsulfonsäure wird die Lösung auf einen pH-Wert von 5,5 eingestellt. Das Natriumsalz wird durch Sprühtrocknung aus der wäßrigen Lösung isoliert.

Zur Beurteilung der Farbe des erhaltenen Natriumsalzes wird die Jodfarbzahl gemäß DIN 53 403 einer 50 gew.%igen wäßrigen Lösung ermittelt.

Beispiel 2

100 Teile Wasser, 100 Teile Acyloxibenzolsulfonsäure und 50 gew.%ige wäßrige Natronlauge läßt man gleichzeitig in einen Kolben einlaufen unter intensiver Mischung mit einem Rührer. Der Zulauf der Natronlauge wird so geregelt, daß ständig ein pH-Wert von 5,5 eingehalten wird. Die Temperatur der Reaktionsmischung wird durch Kühlung unter 50 °C gehalten. Das erhaltene Natriumsalz der Acyloxibenzolsulfonsäure wird durch Sprühtrocknung aus der wäßrigen Lösung isoliert.

Beispiel 3

Es wird gemäß Beispiel 1 gearbeitet, wobei man jedoch dem vorgelegten Wasser 1 Gew.% Natriumdihydrogenphosphat zugibt. Durch Sprühtrocknung wird ein Natriumsalz erhalten, dessen Farbe deutlich heller ist als die Farbe eines Natriumsalzes, das ohne Gegenwart von Natriumdihydrogenphosphat gewonnen wurde.

Beispiel 4

Das gleiche Ergebnis wie in Beispiel 3 wird erhalten, wenn man bei kontinuierlicher Arbeitsweise gemäß Beispiel 2 dem Wasser 1 Gew.% Natriumdihydrogenphosphat zusetzt.

Nach diesen allgemeinen Vorschriften wurden die Natriumsalze der 2-Ethyl-hexanoyl-, n-Octanoyl-, Isononanoyl- (3,5,5-Trimethylhexanoyl-), Oleoyl- und Stearoyloxibenzolsulfonsäure hergestellt.

Beispiel 5

100 Teile Isononanoyloxibenzolsulfonsäure läßt man zu 100 Teilen Wasser zulaufen. Gleichzeitig wird 50 gew.%ige wäßrige Natronlauge so zugetropft, daß ein pH-Wert von 4,0 eingehalten wird. Die Temperatur der Reaktionsmischung wird bei 40 °C gehalten. Die Lösung wird insgesamt 5 h bei 40 °C gerührt. Dann wird mit 50 gew.%iger wäßriger Natronlauge auf einen pH-Wert von 5,5 eingestellt. Das Natriumsalz wird durch Sprühtrocknung aus der Lösung isoliert.

Vergleichsbeispiel A

Man läßt 100 Teile Isononanoyloxibenzolsulfonsäure in 100 Teile Wasser zulaufen. Gleichzeitig wird 50 gew.%ige wäßrige Natronlauge so zugetropft, daß ein pH-Wert von 2,0 eingehalten wird. Die Temperatur der Reaktionsmischung wird bei 40 °C gehalten. Die Lösung wird insgesamt 5 h bei 40 °C gerührt. Dann wird mit 50 gew.%iger wäßriger Natronlauge auf einen pH-Wert von 5,5 gestellt. Das Natriumsalz wird durch Sprühtrocknung aus der Lösung isoliert.

Vergleichsbeispiel B

Es wird gemäß A gearbeitet, jedoch ein pH-Wert von 7,5 eingehalten.

Vergleichsbeispiel C

Es wird gemäß A gearbeitet, jedoch ein pH-Wert von 8,0 eingehalten.

Tabelle 1

| Beispiel | pH-Wert während der 5stündigen Rührzeit | %-Gehalt an Acyloxibenzolsulfonat, bezogen auf Beispiel 5 (Zweiphasentitration nach Epton) |
|---|---|---|
| 5 | 4,0 | 100 |
| A | 2,0 | 68,3 |
| B | 7,5 | 92,1 |
| C | 8,0 | 91,0 |

Der angegebene Estergehalt, der bei einem erfindungsgemäß hergestellten Salz (Beispiel 5) 100% gesetzt wird, drückt eine relative Gehaltsabnahme aus. Tatsächlich werden bei der erfindungsgemässen Arbeitsweise etwa 1 bis 2% Acylverbindung verseift.

Die Tabelle zeigt gegenüber dem erfindungsgemäßen pH-Wertbereich die erhöhte Hydrolyse des Acyloxibenzolsulfonats bei niedrigeren oder höheren pH-Werten.

Tabelle 2: Jodfarbzahlen nach DIN 53 403

| Herstellung von Isononanoyloxibenzolsulfonsäure-Natrium-Salz unter Zusatz von 1 Gew.% von ... | Jodfarbzahl (DIN 53 403) einer 50%igen (m/m)-Lösung |
|---|---|
| Ohne Zusatz | 30 |
| Natriumdihydrogenphosphat | 10 |
| Dinatriumtartrat | 10 |
| Natriumhydrogentartrat | 10 |
| Hypophosphorige Säure | 10 |
| Trinatrium-polyphosphat | 15 |
| Nitrilotriessigsäure | 15 |
| Ethylendiamintetraessigsäure | 15 |
| Diethylentriaminpentaessigsäure | 10 |
| Hydroxiethylethylendiamintriessigsäure | 10 |
| Acrylsäure-Maleinsäure-Copolymeres | 10 |
| Nitrilomethylenphosphonsäure | 10 |

**Patentansprüche**

1. Verfahren zur Herstellung der Alkali- und Erdalkalisalze von Acyloxibenzolsulfonsäuren der Formel I

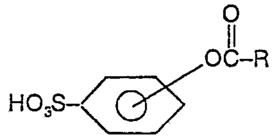

(I),

in der R einen gesättigten oder ungesättigten Alkylrest mit 1 bis 17 C-Atomen, einen Phenylrest, der gegebenenfalls ein- bis zweifach durch einen Alkylrest mit 1 bis 3 C-Atomen, ein Halogenatom, eine Methoxy- oder Nitrogruppe substituiert ist, bedeutet, durch Neutralisation, dadurch gekennzeichnet, daß man eine flüssige Acyloxibenzolsulfonsäure der Formel 1 mit einer wäßrigen Lösung von Alkali- oder Erdalkali in Form des Hydroxids, Carbonats oder Bicarbonats in Wasser bei Temperaturen von 0 bis 60 °C unter guter Durchmischung so zusammenbringt, daß ein pH-Wertbereich von 3,0 bis 5,5 eingehalten wird, und man gegebenenfalls das erhaltene Salz in an sich üblicher Weise aus der wäßrigen Lösung isoliert.

2. Verfahren nach Anspruch 1, dadurch gekennzeichnet, daß die Neutralisation bei Temperaturen von 10 bis 40 °C durchgeführt wird.

3. Verfahren nach Anspruch 1 und/oder 2, dadurch gekennzeichnet, daß die Neutralisation in

Gegenwart von 1 bis 2 Gew.%, bezogen auf die Acyloxibenzolsulfonsäure, eines löslichen Phosphats, Phosphits, Tartrats, eines Komplexbildners für Schwermetallsalze oder eines Polymeren aus Acrylsäure und/oder Maleinsäure durchgeführt wird.

**Claims**

1. A process for the preparation of an alkali metal or alkaline earth metal salt of an acyloxybenzenesulfonic acid of the formula I

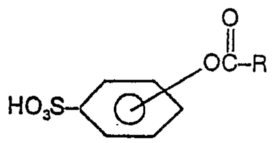

where R is a saturated or unsaturated alkyl radical of 1 to 17 carbon atoms or is phenyl which is unsubstituted or monosubstituted or disubstituted by alkyl of 1 to 3 carbon atoms, halogen, methoxy or nitro, by neutralization, wherein a liquid acyloxybenzenesulfonic acid of the formula I and an aqueous solution of an alkali metal or alkaline earth metal hydroxide, carbonate or bicarbonate are combined in water, at from 0 to 60 °C and while mixing thoroughly, so that the pH is kept at from 3.0 to 5.5, and, if desired, the resulting salt is isolated from the aqueous solution in a conventional manner.

2. A process as claimed in claim 1, wherein the neutralization is carried out at from 10 to 40 °C.

3. A process as claimed in claims 1 and/or 2, wherein the neutralization is carried out in the presence of from 1 to 2% by weight, based on the acyloxybenzenesulfonic acid, of a soluble phosphate, phosphite or tartrate, of a complexing agent for heavy metal salts, or of a polymer of acrylic acid and/or maleic acid.

**Revendications**

1. Procédé de préparation de sels de métaux alcalins et alcalino-terreux d'acides acyloxybenzène-sulfoniques de la formule I

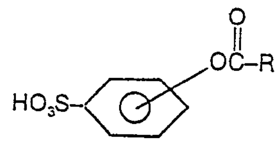

(I),

dans laquelle R désigne un radical alkyle en $C_1$ à $C_{17}$ saturé ou non saturé, un groupe phényle éventuellement mono- ou di-substitué par un radical alkyle en $C_1$ à $C_3$, un atome d'halogène ou un groupe méthoxy ou nitro, par une neutralisation, caractérisé en ce que l'on réunit sous une bonne agitation, entre 0 et 60 °C, un acide acyloxybenzène-sulfonique liquide de la formule I et une solution aqueuse d'un métal alcalin ou alcalino-terreux sous forme d'hydroxyde, de carbonate ou d'hydrogénocarbonate en des proportions telles que le pH se situe entre 3,0 et 5,5, puis l'on isole le cas échéant le sel formé de façon usuelle de la solution aqueuse.

2. Procédé suivant la revendication 1, caractérisé en ce que la neutralisation est réalisée entre 10 et 40 °C.

3. Procédé suivant la revendication 1 et(ou) la revendication 2, caractérisé en ce que la neutralisation est réalisée en présence de 1 à 2% du poids de l'acide acyloxybenzène-sulfonique d'un phosphate, phosphite ou tartrate soluble, d'un complexant (chélatant) des métaux lourds ou d'un polymère de l'acide acrylique et(ou) de l'acide maléique.